# EUROPEAN PATENT APPLICATION

(11) **EP 2 248 535 A1**
(43) Date of publication of application: **10.11.2010**
(21) Application number: 10171629.8
(22) Date of filing: 02.11.1999
(51) Int. Cl.: A61K 39/095, C07K 14/22, A61P 31/04

(54) **Multicomponent meningococcal vaccine**

(30) Priority: 02.11.1998 GB 9823978
(62) Divisional of application: 02025569.1
(71) Applicant: Health Protection Agency, Salisbury, Wiltshire SP4 0JG (GB)
(72) Inventor: Robinson, Andrew, Salisbury, Wiltshire SP4 0JG (GB); Gorringe, Andrew, Salisbury, Wiltshire SP4 0JG (GB); Hudson, Michael John, Salisbury, Wiltshire SP4 0JG (GB); Reddin, Karen Margaret, Salisbury, Wiltshire SP4 0JG (GB)
(74) Representative: Griffin, Philippa Jane

(57) **Abstract**

There is provided a composition comprising *N*. *meningitidis* outer membrane vesicles, wherein said outer membrane vesicles are enriched with at least one antigenic component.

## Description

The present invention relates to a multicomponent vaccine and methods for preparing a multicomponent vaccine that confers protective immunity to a broad spectrum of infection by Gram negative pathogens. In particular the present invention relates to a multicomponent vaccine that provides both passive and active protective immunity to meningococcal disease.

Meningococcal meningitis is a major problem worldwide and in many countries incidence of infection is increasing. *Neisseria meningitidis* is the causative agent of the disease and is also responsible for meningococcal septicaemia, which is associated with rapid onset and high mortality, with around 22% of cases proving fatal.

At present, vaccines directed at providing protective immunity against meningococcal disease provide only limited protection because of the many different strains of *N. meningitidis.* Vaccines based upon the serogroup antigens, the capsular polysaccharides, offer only short lived protection against infection and do not protect against many strains commonly found in North America and Europe. A further drawback of these vaccines is that they provide low levels of protection for children under the age of 2 years, one of the most vulnerable groups that are commonly susceptible to infection.

The meningococcal transferrin receptor is made up of two types of component protein chain, Transferrin binding protein A (TbpA) and TbpB. The receptor complex is believed to be formed from a dimer of TbpA which associates with a single TbpB (Boulton *et al.* (1998)). Epitopes present in TbpA are known to be masked within the interior of the protein (Ala'Aideen (1996)). Vaccines against meningococcal meningitis based on TbpB from one strain alone show some cross reactivity and there is evidence of a cross-reactive immune response in rabbits immunised with TbpB alone (Feirrerós *et al.* (1998)).

It would be of advantage, nevertheless, to provide a vaccine that gives a broader range of protective immunity to infection from a wider spectrum of strains of *N*. *meningitidis.* It would be of further advantage to provide a vaccine that confers protective immunity to infants as well as adults and whose protection is long term. It would also be of advantage to provide a vaccine that protects against sub-clinical infection, i.e. where symptoms of meningococcal infection are not immediately apparent that the infected individual may act as a carrier of the pathogen.

It is an object of the present invention to provide compositions containing Tbps, and vaccines based thereon, that meet or at least ameliorate the disadvantages in the art. In particular, it is an object of the invention to provide a vaccine composition that consistently and reliably induces protective immunity to meningococcal infection.

Accordingly, a first aspect of the present invention provides a composition comprising both transferrin binding proteins A (TbpA) and B (TbpB), suitably in a molar ratio of about 2:1 (TbpA:TbpB). In a preferred embodiment of the present invention the molar ratio of TbpA to TbpB is 2:1.

The composition may be combined with a pharmaceutically acceptable carrier - for example the adjuvant alum although any carrier suitable for oral, intravenous, subcutaneous, intraperitoneal or any other route of administration is suitable - to produce a pharmaceutical composition for treatment of meningococcal disease.

The present invention thus provides for a vaccine comprising both TbpA + B proteins, preferably with a molar ratio of between 1.8 and 2.2 molecules of TbpA to one molecule of TbpB, more preferably 2 molecules of TbpA to one of TbpB. This particular combination of components, surprisingly, can provide higher protective immunity to meningococcal infection, compared to vaccination with TbpB alone. In a specific embodiment of the invention, described in more detail below, a 1:1 combination of A:B is more protective against challenge than B alone. This is surprising as TbpA has previously been considered to be non-protective. The present results differ from this established view with some experiments (described in more detail below) showing that, when administered as a vaccine, TbpA is also able to provide protective immunity to meningococcal infection. However, the present results most strikingly demonstrate the consistent performance of vaccines that comprise both Tbps A and B compared to those comprising Tbp A or B alone. It is this lack of variability between compositions and the consistently high level of protection to infection induced in response to vaccination with Tbp A+B, that enables the compositions of the invention to demonstrate significant advantage over the vaccines of the prior art.

Transferrin binding proteins are known to be located on the outer membranes of a number of Gram negative bacteria such as *N. meningitidis.* Formulations of the composition of the present invention with conventional carriers or adjuvants provide a composition for treatment of infection by these bacteria.

It is an advantage that following administration of a composition according to the present invention antibodies may be raised against epitopes that consist of sequences from TbpA and TbpB in juxtaposition. Thus, the immune response obtainable using such a composition may be improved compared with that from prior art vaccine compositions which comprise only one component of the Tbp complex and in which the full range of potential Tbp epitopes are unavailable. It is a further option in the present invention for one Tbp subunit component of the TbpA + B complex to be from a first strain of *N. meningitidis* and another from a second strain different from the first. For example, the TbpA dimer is taken from the first strain and the TbpB is from the second. The TbpA and TbpB proteins may be selected independently from strains K454, H44/76 and B16B6. In all aspects of the invention the Tbps can be directly isolated from the bacterial source or can be produced by recombinant methods commonly known in the art. Combinations of proteins from other strains are also envisaged, and the combining of components from different strains of bacteria offers the potential for providing an individual with a broader spectrum of protection against meningococcal infection. It is further optional for a composition or vaccine of the invention to contain a mixture of A proteins from different strains or a mixture of B proteins from different strains, broadening further the potential spectrum of protection conferred by the invention. A still further option is for Tbps to be obtained from or derived from other bacteria, including *N.gonorrhoeae, N.lactamica* and *Moraxella catarrhalis.*

In the present invention, the term "transferrin binding protein" or "Tbp" refers to a protein which either alone binds to transferrin or can be part of a complex of proteins that binds transferrin. The term also embraces fragments, variants and derivatives of such a protein provided that antibodies raised against the fragment, variant or derivative bind the protein. Thus, TbpA and TbpB either dissociated or associated into a complex are considered to be Tbp. Moreover, mutants, fusion proteins or fragments of either TbpA or B or other derivatives of the TbpA + B complex with a common antigenic identity are also considered to be represented by the term Tbp in the present invention.

A second aspect of the invention provides a composition comprising a complex of two TbpAs and one TbpB. The proteins are thus held together in the ratio seen in the native receptor. The individual proteins may be linked, for example, by hydrogen bonds or covalent bonds. In the latter case, each TbpA is covalently linked to the TbpA, either directly or indirectly. In a preferred embodiment, the complex of TbpA and TbpB assumes a native configuration.

A native TbpA + B complex may be isolated and purified from *N. meningitidis.* Alternatively, the invention also provides for synthesis of recombinant Tbp protein followed by assembly of the TbpA + B complex *in vitro.* The TbpA + B complex may be formed by admixture, or may be crosslinked by physical (e.g. UV radiation) or chemical methods known to the art, resulting in a combination of Tbps that will remain together and can not dissociate from each other. In a further example, a single chain recombinant protein comprising two TbpA sequences, preferably in the form of the TbpA dimer, is then covalently linked with TbpB protein to form a complete TbpA + B complex *in vitro.* Another example of the invention in use provides that TbpA and B are mutated so as to introduce cysteine residues that facilitate the formation of dliulphide bridges between the TbpA and TbpB subunits, so as to obtain a covalently bound complex.

In preparation of a recombinant protein TbpA and TbpB genes may also be truncated so that only those domains known to contribute to the antigenicity of the protein are incorporated in the Tbp complex.

In some compositions of the invention the TbpA + B complex is able to act as a transferrin receptor and binds to human transferrin. In other compositions of the invention the TbpA + B complex is non-functional in the sense that it does not bind transferrin, but it nevertheless provides an antigenic component that elicits an appropriate immune response.

A third aspect of the invention provides for a composition comprising a Tbp and *N. meningitidis* outer membrane vesicles. An advantage of this composition is that when administered to a vaccinee or patient it presents a different combination of *N. meningitidis* antigens, and particularly antigens that are in a formation substantially as present on the membrane of live infecting organisms. The combination offers the potential for a more effective protection against infection or a broader spectrum of protection than existing vaccines. Known methods of outer membrane vesicle isolation, such as by desoxycholate treatment, are suitable for preparation of compositions of the invention. In various preferred embodiments the Tbp is Tbp A, Tbp B or TbpA + B either in a native complex or in dissociated form.

The outer membrane vesicles may further be pretreated *in vitro* with Tbp so as to enrich the vesicle membrane with Tbp. By "enrich" and like terms, we refer to an outer membrane vesicle to which has been added Tbps so as to increase the concentration or density of Tbps in that vesicle. Preferably, the enrichment results in an outer membrane vesicle having an increased number of transferrin receptors located in the membrane, due to an increased concentration of TbpA and TbpB following their addition to the vesicle and their association into receptors or receptor-like structures. A particular advantage of such embodiments of the invention is that the Tbps, regarded as key antigenic components of the vaccine, are presented in a highly antigenic environment that closely mimics the environment in which transferrin receptors are presented on live, infecting bacteria.

As mentioned previously, the Tbp components of the composition need not be wild-type Tbps. They can be made recombinantly, and in so doing sequence alterations may be introduced. In one typical example of the invention, recombinant TbpB is modified so as to comprise a membrane binding domain. A preferred membrane spanning domain is a hydrophobic alpha helical region added to either the N or C terminus of the Tbp B protein. However, a membrane anchoring region need not only be an alpha helix, addition of a fatty acid or lipid chain would also facilitate membrane anchoring. In fact, wild type TbpB is believed to anchored to the bacterial outer membrane via such a lipid chain anchor. In a further example of the invention in use, the outer membrane vesicles are pretreated *in vitro* with membrane binding recombinant TbpB so as to enrich the vesicle membrane with TbpB. OMVs enriched with Tbps are additionally generated by inducing high levels of Tbp expression in *N.meningitidis* and then isolating OMVs via one of the methods described previously. This latter method is typically achieved by transforming the *N.meningitidis* host with a suitable expression vector into which has been inserted a gene or genes encoding the Tbps of choice. Suitable expression vectors for use in neisserial species include the plasmid pMGC10 (Nassif *et al.* (1991)).

It is preferred that the composition of the present invention comprises outer membrane Vesicles and TbpA + B complexes isolated from a range of different strains of *N. meningitidis.* Other preferred compositions of the invention comprise other *N. meningitidis* proteins including surface antigens, periplasmic proteins, superoxide dismutase and glycoproteins.

The composition of the third aspect of the invention may instead of or in addition to outer membrane vesicles comprise one or more liposomes, each liposome including TbpA and / or TbpB preferably including TbpA and TbpB associated into a receptor or into a receptor-like complex. Thus a further means of presenting the transferrin receptor antigens is provided.

In a further embodiment of the third aspect of the invention, the composition comprises 22 kD antigen (Neisserial surface protein A (NspA)) as well as or instead of outer membrane vesicles. NspA and its preparation are described by Martin *et al*. 1997.

A fourth aspect of the invention provides for a vaccine comprising a composition of the invention as described above. A vaccine of the invention may also comprise antibodies to Tbp and thus provide a level of passive immunity to bacterial infection.

A fifth aspect of the invention provides for a method of manufacturing a composition that comprises combining TbpA, TbpB and *N. meningitidis* outer membrane vesicles with a pharmaceutically acceptable carrier. It is preferred that the molar ratio of TbpA to TbpB is about 2:1. The outer membrane vesicles can be pretreated *in vitro* with native TbpA + B so as to enrich the vesicle membrane with Tbp complex. However, the outer membrane vesicles may also be pretreated with other protein components so as to enrich them for these antigenic components also. The outer membrane vesicles may also be pretreated with antigenic proteins and proteoglycans from several different strains of *N. meningitidis.*

A further aspect of the invention provides for a composition comprising a Tbp and a Cu,Zn-Superoxide dismutase (Cu,Zn-SOD).

Cu,Zn-Superoxide Dismutase (Cu,Zn-SOD) is an metalloenzyme found in many prokaryotic and eukaryotic organisms. It catalyses the reduction of the superoxide radical anion, O₂, to hydrogen peroxide and molecular oxygen, thus playing an important role in the removal of cytotoxic free radicals from the organism. In bacteria Cu,Zn-SODs have been identified in the periplasm of a number of Gram negative species including *N. meningitidis.* The enzyme can exist as a dimer or a monomer, and accordingly in a preferred embodiment the present invention provides for a composition comprising a Tbp and a Cu,Zn-SOD of the dimeric type.

As mentioned previously with regard to the use of the term "Tbp", the Cu,Zn-SOD of the present invention is also considered to encompass fragments, variants and derivatives of such a protein provided that antibodies raised against the fragment, variant or derivative bind the wild type Cu,Zn-SOD.

In examples of the invention in use, compositions are provided that comprise a Cu,Zn-SOD and either a TbpA, a TbpB or a TbpA + B complex. In the latter example it is preferred that the molar ratio of TbpA to Tbp B is between 1.8 and 2.2, with the most suitable compositions having a ratio of 2:1 (TbpA:TbpB). In further compositions of the invention the Tbps are from a different strain of *N.meningitidis* to that of the Cu,Zn-SOD, thus facilitating the formation of a broader spectrum of immune response to meningococcal infection.

The invention also provides for compositions wherein the Tbps and Cu,Zn-SOD are from different bacterial species, typically different Gram negative species. Such compositions thereby allow an even broader spectrum of immune response to be elicited when administered as a vaccine. Typical compositions comprise a neisserial TbpA+B complex as well as a monomeric Cu,Zn-SOD - from *E.coli* for example - and optionally a further dimeric Cu,Zn-SOD - for example from *Haemophilus parainfluenzae.*

Other aspects of the invention provide for methods of manufacturing compositions that provide protective immunity to Gram negative bacterial infection, comprising combining a covalently linked complex of TbpA and TbpB with either or both of *N. meningitidis* outer membrane vesicles and a Cu,Zn-SOD plus a pharmaceutically acceptable carrier.

A further aspect provides for use of Tbps A and B in the manufacture of a medicament for human vaccination. It is preferred that such a medicament is suitable for vaccination against meningococcal infection, although some compositions of the invention provide broad spectrum protection to infection from a wider range of bacterial pathogens.

The embodiments of the invention are discussed in more detail by means of the Example described below. The results referred to in the Example are illustrated by the accompanying drawings, in which:
Fig.1 shows immunisation of mice with TbpA+B and outer membrane vesicles;
Fig.2 shows immunisation of mice with TbpA + B;
Fig.3 shows protection of mice against IP infection after immunisation with TbpA + B, isolated TbpA or isolated TbpB;
Figs.4A and 4B show protection of mice against meningococcal infection following immunisation with either neisserial TbpA + B, TbpB or TbpA (nTbps) or recombinant Tbps (rTbps);
Figs.5A and 5B show protection of mice against challenges of 10⁶ and 10⁷ organisms/mouse of *N.meningitidis* strain K454 respectively, following immunisation with recombinant TbpA + B, TbpB or TbpA;
Fig.6 shows protection against challenge with heterologous serogroup; and
Fig.7 shows protection against challenge with B16B6

### Example

### Mouse protective data

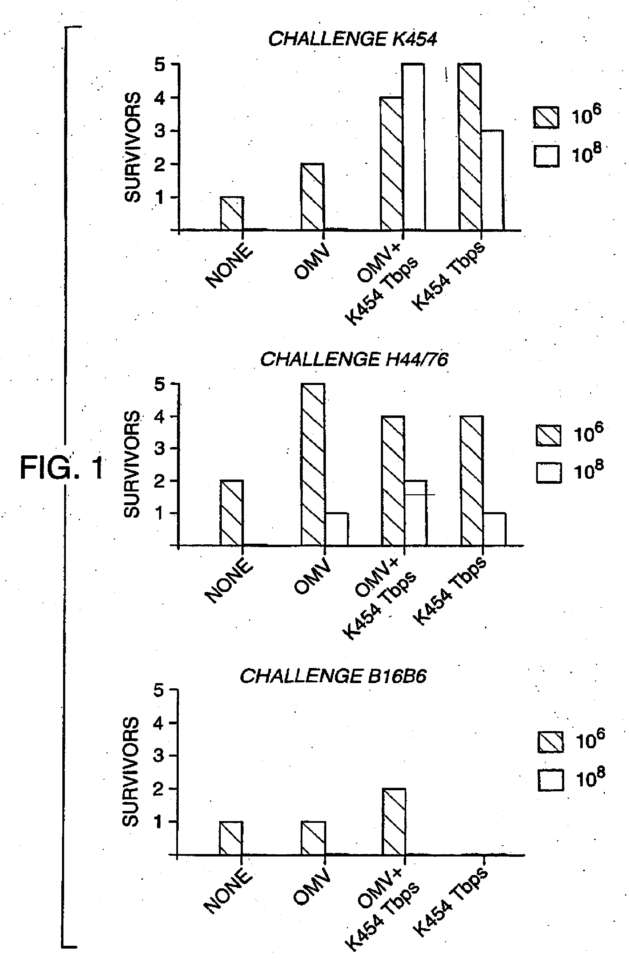

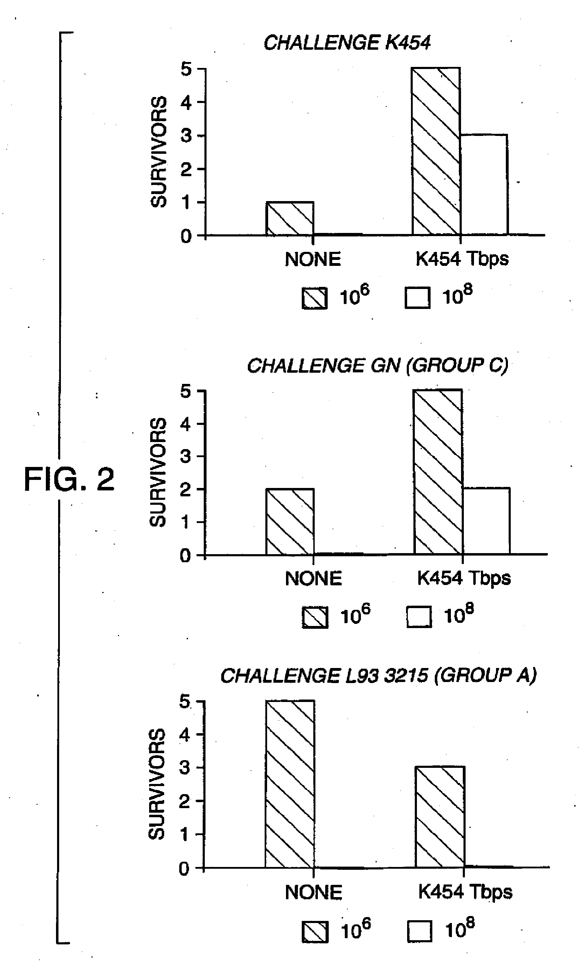

Mice (CAMR-NIH) were immunised with Tbps and /or outer membrane vesicles and challenged with either the homologous or a heterologous meningococcal strain. Survivors per group of mice immunised with Tbps from strain K454 and the outer membrane vesicle vaccine following challenge by strain K454 are shown in Fig. 1. Compared to Tbps, the outer membrane vesicle vaccine gave reduced protection but this may be because it is produced from a different group B strain (H44/76). Animals immunised with TbpA + B isolated from strain K454 were also protected against challenge with other serogroup B organisms (Fig. 1), with greater protection seen with the homologous strain and strains expressing a TbpB with a similar molecular weight. Little or no protection was observed against challenge with meningococci possessing TbpB with a very different molecular weight (strain B16B6). With the heterologous challenge strains, there is a slightly greater number survivors in the groups vaccinated with the combination of Tbps + outer membrane vesicles. However, the numbers involved are small and no definite conclusions can be reached. It is interesting to observe that mice immunised with TbpA + B from strain K454 were also protected against infection with a serogroup C but not a serogroup A strain (Fig. 2).

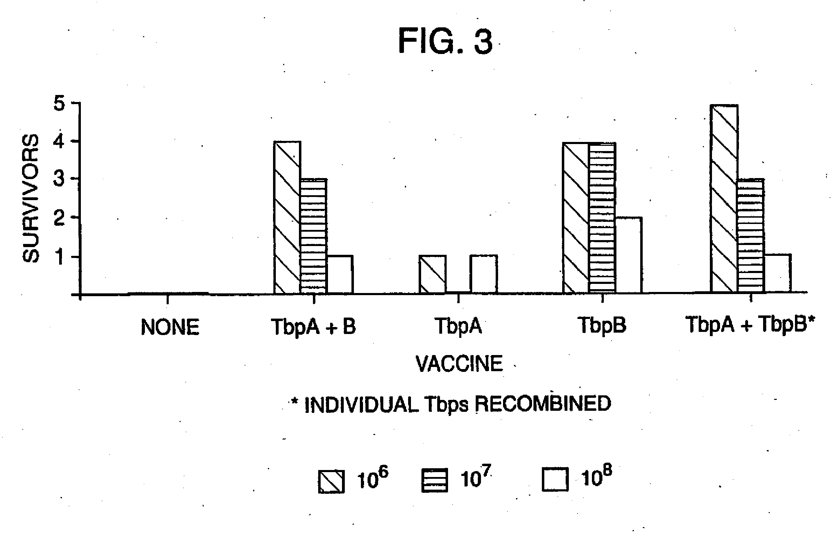

Protection against challenge with strain K454 with mice immunised with co-purified TbpA + B and isolated TbpA and TbpB is shown in Fig. 3. It can be seen that TbpB is the predominant antigen responsible for protection, with little protection afforded by TbpA alone.

### Recombinant TbpA and TbpB

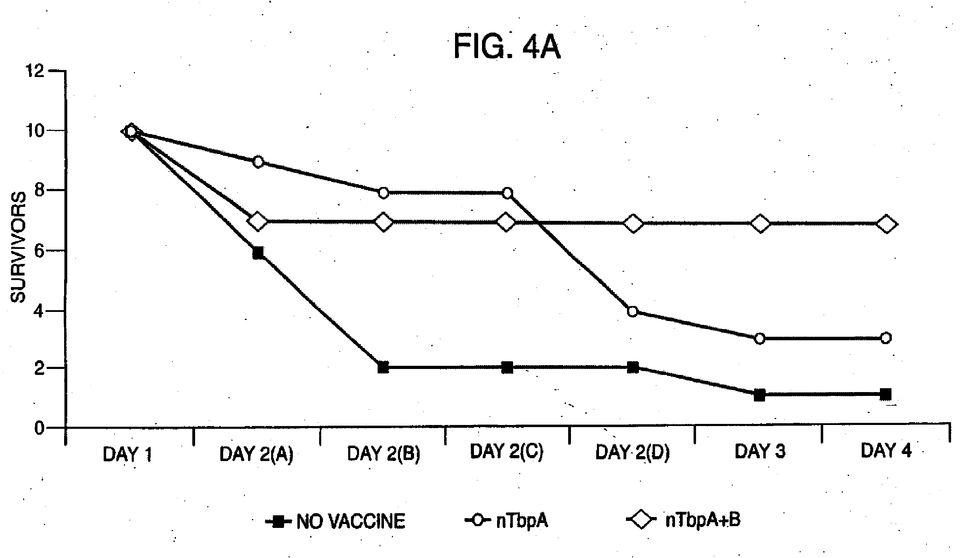

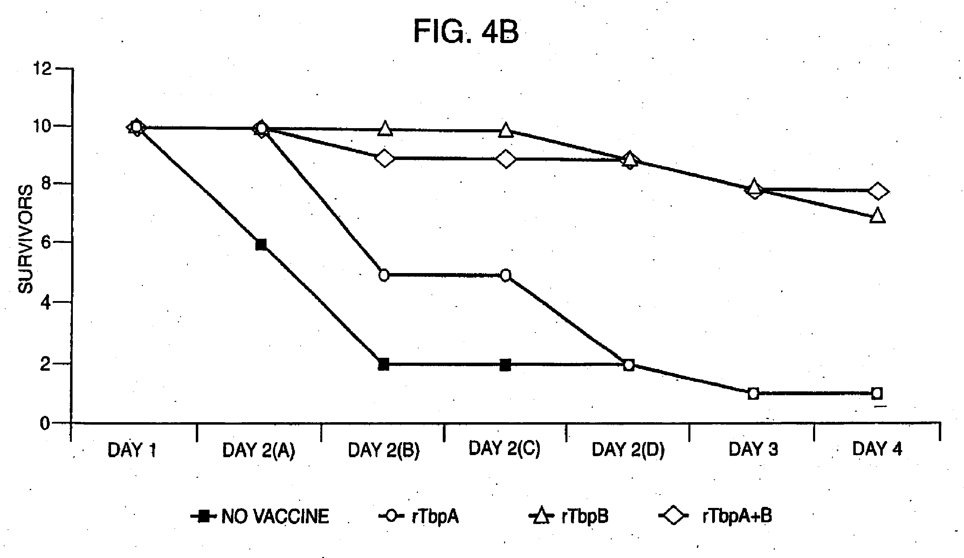

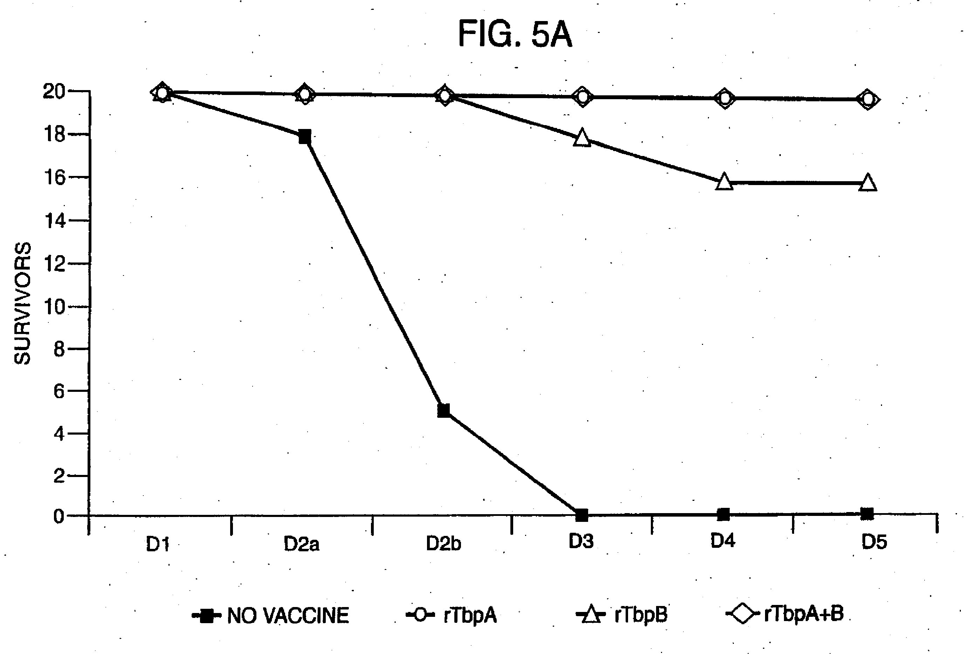

TbpA and TbpB from *N. meningitidis* strain K454 were cloned and overexpressed in *E. coli.* The proteins were purified using affinity chromatography and used to determine their protective potency in a mouse model of meningococcal disease. Recombinant Tbps showed equivalent protection to that provided by Tbps isolated from iron-stressed *N. meningitidis* (Figs. 4A and 4B). These recombinant Tbps were also utilised in two further larger IP challenge experiments (Figs. 5A-B).

The strong and consistent protective potency of Tbps against mouse I.P. infection with *N. meningitidis* is probably the most compelling evidence for their vaccine potential.

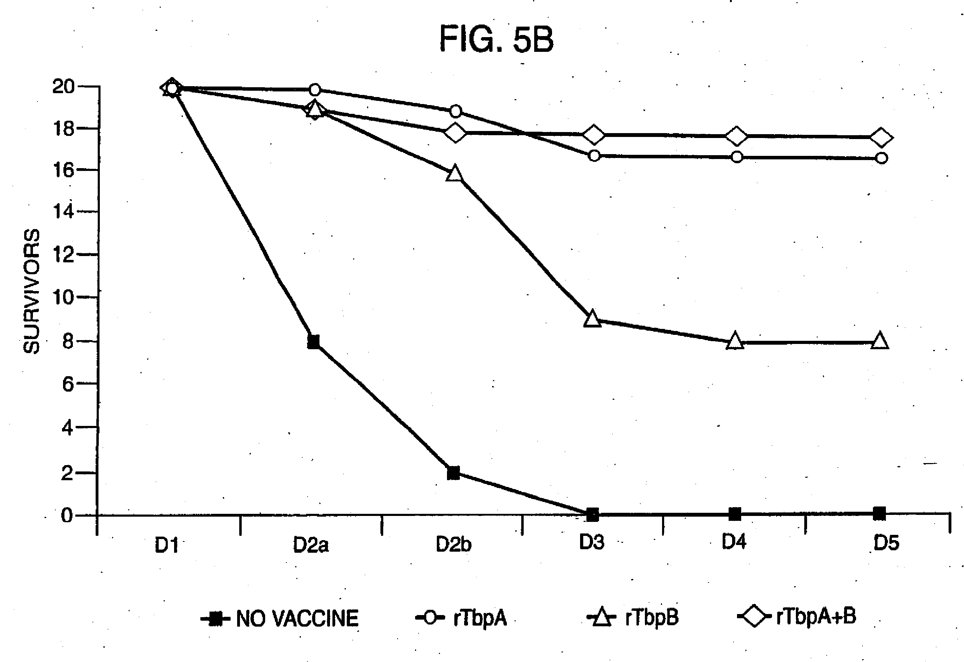

Interestingly, the results shown in Figs. 4B and 5A-B, although arising from experiments of similar design, show the surprising variability associated with vaccine compositions that depend solely upon either TbpA or TbpB alone. In Fig. 4B the recombinant TbpA composition displayed low levels of protection compared to TbpB or TbpA + B. However, in Figs. 5A and 5B the recombinant TbpA vaccine composition that showed high levels of protection comparable to the TbpA + B complex and the TbpB alone composition demonstrated poor protection to infection. This latter result is completely contrary to the teaching of the prior art.

### Human immune response to TbpA and TbpB in convalescent sera

We have undertaken a number of studies looking at the antibody response in humans to TbpA and TbpB following meningococcal disease. The general conclusions are that both TbpA and TbpB are expressed during meningococcal disease and that an immune response is raised against them. The response is functional (opsonic) and is more cross-reactive between different meningococcal strains than is the response induced by immunisation of animals with Tbps. The immune response to TbpA appears to be stronger and more cross-reactive than that to TbpB, confirming the importance of the vaccine potential of TbpA.

### TbpA and TbpB form a transferrin receptor

Our structural studies indicate that the transferrin receptor on the meningococcal surface consists of two TbpA molecules and one TbpB molecule that act together.

### Effect of a Vaccine containing A + B Tbps

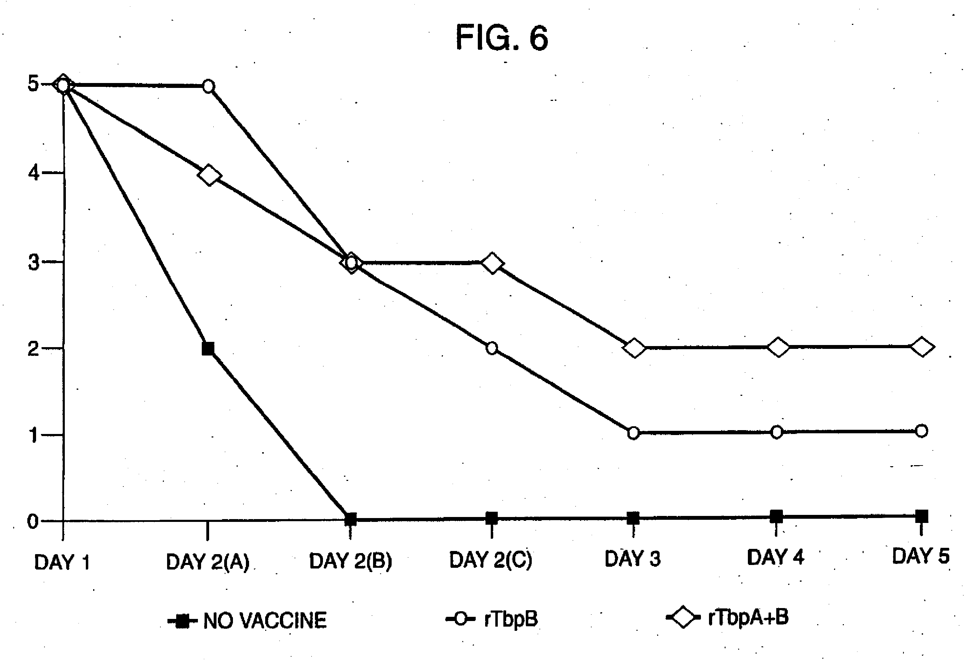

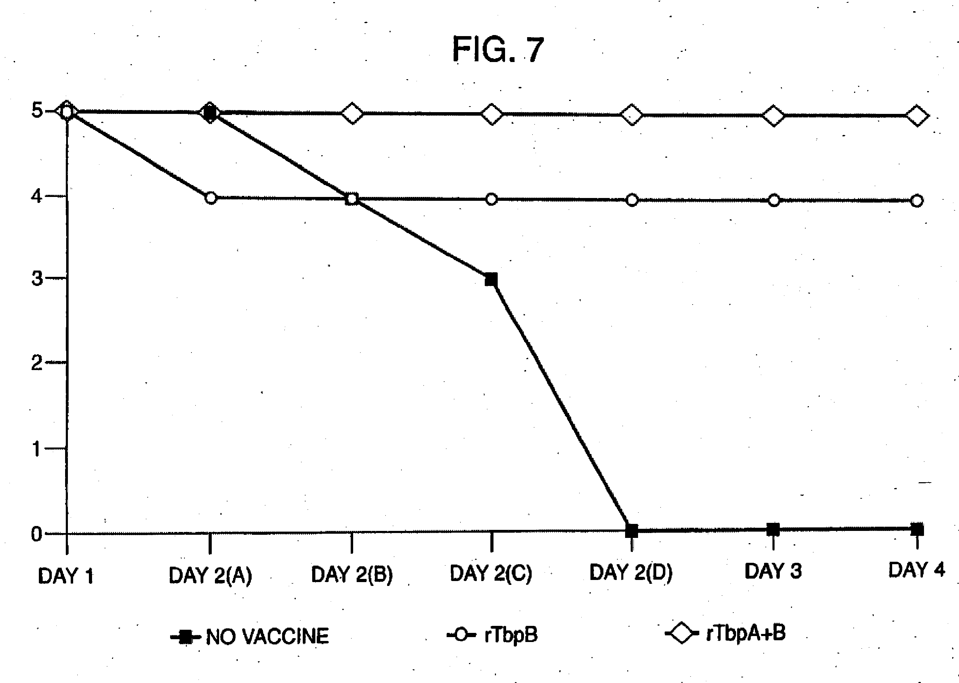

We carried out further tests of the efficacy of recombinant TbpB versus recombinant TbpA + B containing vaccine formulations against challenge from *N.meningitidis* strains L91 705 and B16B6, the results of which are illustrated in Figs 6 and 7 respectively. In both cases some improved protection was conferred by A + B compared to B alone.

### References:

Ala'Aldeen J. (1996) Med. Microbiol vol 44, pp237-243.
Boulton et al. (1998) Biochem .J. vol.334, pp269-273.
Feirrerós et al. (1998) Rev. in Med. Microbiol. vol 9, No.1., pp29-37.
Gorringe et al. (1996) 10th Pathogenic Neisseria Conference, poster 46 abstract.
Martin et al. (1997) J Exp Med, vol.185 no. 7 pp1173-1181.
Nassif et al. (1991) J Bacteriol, vol.173 no. 7 pp2147-54.

## Claims

1. A composition comprising *N. meningitidis* outer membrane vesicles, wherein said outer membrane vesicles are enriched with at least one antigenic component.

2. A composition according to Claim 1, wherein said antigenic component is an *N. meningitidis* antigenic protein, such as an *N. meningitidis* antigenic proteoglycan, or an *N.meningitidis* protein selected from a surface antigen, a periplasmic protein, a superoxide dismutase, or a glycoprotein.

3. A composition according to any of Claims 1 or 2, wherein said outer membrane vesicles are from a first strain of *N. meningitidis* and said antigenic component is from a second strain of *N. meningitidis* different from the first, optionally further comprising a pharmaceutically acceptable carrier.

4. A composition according to any previous Claim, wherein said outer membrane vesicles are enriched with a plurality of antigenic components from different strains of *N. meningitidis.*

5. A composition according to any previous Claim, wherein said outer membrane vesicles comprise a mixture of outer membrane vesicles from different strains of *N. meningitidis.*

6. A composition according to Claim 2, wherein said antigenic component is selected from Cu,Zn-superoxide dismutase or Neisserial surface protein A (NspA).

7. A vaccine composition comprising: (i) outer membrane vesicles from a first strain of *N. meningitidis,* (ii) an antigenic component from a second strain of *N. meningitidis* different from the first, and (iii) a pharmaceutically acceptable carrier; preferably wherein said antigenic component is an *N. meningitidis* protein selected from a surface antigen, a periplasmic protein, a superoxide dismutase, or a glycoprotein.

8. A method of manufacture of a composition, comprising:
(a) extracting an antigenic component from an outer membrane of a bacteria, and preparing an aqueous solution of said antigenic component;
(b) extracting outer membrane vesicles from a culture of *N.meningitidis,* and preparing an aqueous solution of said outer membrane vesicles;
(c) obtaining a pharmaceutically acceptable carrier; and
(d) admixing the solution prepared in step (a), the solution prepared in step (b) and the carrier obtained in step (c).

9. A method according to Claim 8, wherein said antigenic component is from a first strain of *N. meningitidis,* and said outer membrane vesicles are from a second strain of *N. meningitidis* different from the first, preferably wherein said strains of *N. meningitidis* are selected from K454; H44/76; or B16B6.

10. A method of manufacture of a composition, comprising:
(a) recombinantly expressing a DNA that encodes an antigenic component in a bacteria, such as a strain of *N. meningitidis*;
(b) extracting said antigenic component from the outer membrane of said bacteria, and preparing an aqueous solution of said antigenic component;
(c) extracting outer membrane vesicles from a culture of *N. meningitidis,* and preparing an aqueous solution of said outer membrane vesicles;
(d) obtaining a pharmaceutically acceptable carrier; and
(e) admixing the solution prepared in step (b), the solution prepared in step (c) and the carrier obtained in step (d).

11. A method of manufacture of a composition, comprising:
(a) recombinantly expressing a DNA that encodes an antigenic component in *N. meningitidis*;
(b) extracting outer membrane vesicles from said *N. meningitidis,* and preparing an aqueous solution of said outer membrane vesicles, wherein said outer membrane vesicles comprise said antigenic component;
(c) obtaining a pharmaceutically acceptable carrier; and
(d) admixing the solution prepared in step (b), with the carrier obtained in step (c).

12. A method according to any of Claims 8-11, wherein said antigenic component is an *N. meningitidis* antigenic protein, such as an *N. meningitidis* antigenic proteoglycan or an *N. meningitidis* protein selected from a surface antigen, a periplasmic protein, a superoxide dismutase, or a glycoprotein, preferably wherein said antigenic component is either Cu,Zn-superoxide dismutase; or Neisserial surface protein A (NspA).

13. A composition comprising *N. meningitidis* outer membrane vesicles, wherein said outer membrane vesicles are enriched with at least one antigenic component, for use in preventing meningococcal infection in an animal, such as a human.

14. A composition for use according to Claim 13, wherein said antigenic component is an *N. meningitidis* antigenic protein, such as an *N. meningitidis* protein selected from a surface antigen, a periplasmic protein, a superoxide dismutase, and a glycoprotein.

15. A composition for use according to Claim 13 or 14, wherein said outer membrane vesicles are from a first strain of *N. meningitidis* and said antigenic component is from a second strain of *N. meningitidis* different from the first.
